# EUROPÄISCHE PATENTSCHRIFT

(11) **EP 1 594 886 B1**
(45) Veröffentlichungstag und Bekanntmachung des Hinweises auf die Patenterteilung: **27.08.2008**
(21) Anmeldenummer: 04712555.4
(22) Anmeldetag: 19.02.2004
(51) Int. Cl.: C07J 63/00, A61P 35/00

(54) **ANTITUMOR WIRKSAME 2-SUBSTITUIERTE D-HOMOESTRA-1,3,5(10)-TRIEN-3-YL SULFAMATE**
ANTITUMORAL D-HOMOESTRA-1, 3, 5 (10)-TRIEN-3-YL 2-SUBSTITUTED SULFAMATES
SULFAMATES D-HOMOESTRA-1,3,5(10)-TRIENE-3-YLE 2-SUBSTITUES A EFFET ANTITUMEUR

(30) Priorität: 19.02.2003 DE 10307103
(43) Veröffentlichungstag der Anmeldung: 16.11.2005
(73) Patentinhaber: Sterix Limited, Slough, Berkshire SL1 3XE (GB)
(72) Erfinder: HILLISCH, Alexander, 42553 Velbert (DE); PETERS, Olaf, 99891 Tabarz (DE); GEGE, Christian, 89584 Ehingen/Donau (DE); SIEMEISTER, Gerhard, 13503 Berlin (DE); UNGER, Eberhard, 07751 Cospeda (DE); MENZENBACH, Bernd, 07743 Jena (DE)
(74) Vertreter: Alcock, David
(86) Internationale Anmeldenummer: PCT/EP2004/001629
(87) Internationale Veröffentlichungsnummer: WO 2004/074309

(56) Entgegenhaltungen:
- WO-A-01/30803
- US-A- 6 046 186

## Beschreibung

Die Erfindung betrifft 2-Substituierte D-Homöstra-1,3,5(10)-trien-3-yl-sulfamate, sie enthaltende Zusammensetzungen und ihre Verwendung zur Herstellung pharmazeutischer Mittel mit antitumoraler Aktivität.

Mikrotubuli sind Organellen, die in den meisten eukaryotischen Zellen vorkommen und dort eine Reihe von Funktionen wie Mitose, intrazelluläre Bewegungen, Zellwanderung und die Ausprägung der Zellform übemhemen. Mikrotubuli sind Polymere, die aus Tubulin, das seinerseits ein Dimer aus einer α- und einer β-Einheit darstellt. Diese Heterodimere binden zwei Moleküle Guanosintriphosphat (GTP), wobei eines der GTP fest gebunden und das austauschbar ist. Die Heterodimere polymerisieren in einer Kopf-Schwanz-Anordnung zu fadenförmigen Makromolekülen, den sogenannten Protofilamenten, die sich ihrerseits zu röhrenförmigen Organellen, den Mikrotubuli zusammenlagem.

Mikrotubuli unterliegen einem ständigen Auf- und Abbau. Das Gleichgewicht zwischen Wachstum und Abbau hängt von der Verfügbarkeit neuer GTP-Tubulin-Untereinheiten und der Hydrolysegeschwindigkeit des zweiten gebundenen GTP's ab. Am Plus-Ende werden neue Untereinheiten angebaut, wogegen am Minus-Ende Untereinheiten abdiffundieren.

Es ist bekannt, dass zytotoxische Substanzen wie Colchicin, Vinblastin, Vincristin, Taxol, Ephothilone, Podophyllotoxin, Steganicin, Combretastatin und 2-Methoxyestradiol den Auf- bzw. Abbau der Mikrotubuli (Tubulinpolymerisation und - Tubulindepolymerisation) beeinflussen und somit in der Lage sind die Zellteilung phasenspezifische zu beeinflussen. Dies betrifft vor allem schnell wachsende, neoplastische Zellen, deren Wachstum durch intrazelluläre Regelmechanismen weitgehend unbeeinflusst ist. Wirkstoffe dieser Art sind prinzipiell geeignet zur Behandlung maligner Tumoren.

Fotsis et. al. Nature 1994 368, 237-239 berichten darüber, dass 2-Methoxyestradiol das Tumorwachstum und die Angiogenese hemmt.

Cushman et. al. J. Med. Chem. 1995 38, 2041-2049 untersuchen die zytotoxische sowie die tubulinpolymerisationshemmende Wirkung von 2-Methoxyestradiol, und berichten in J. Med. Chem. 1997, 40, 2323-2334 darüber, dass 2-Alkoxy-6-oximinoestradiol-Derivate die Tubulinpolymerisation sowie die Bindung von [³H]-Cholchicin an Tubulin hemmen. Die hier genannten 2-Alkoxy-6-oximinoestradiol-Derivate zeigen bzgl. der Hemmung der Tubulinpolymerisation vergleichbare Aktivität wie 2-Ethoxyestradiol, das eine höhere Aktivität als 2-Methoxyestradiol aufweist.

Steroid-3-sulfamate werden andererseits in der Literatur als Hemmstoffe der Steroidsulfatase beschrieben:

WO93/05064 betrifft u.a. Verbindungen der Formel 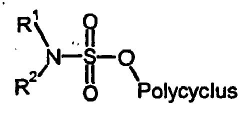 wobei R¹ und R² jeweils unabhängig Wasserstoff oder Methylgruppe bedeuten, mit der Maßgabe, dass mindestens einer der Reste R¹ und R² ein H-Atom ist, und der Rest-O-Polycyclus ein 3-Sterol ist, dessen Sulfatester durch ein Enzym mit Steroidsulfatase-Aktivität hydrolisierbar ist. In der 2-Stellung des Steroidgerüstes spezifisch substituierte Verbindungen sind nicht explizit offenbart.

US 6,011,024 beruht auf der WO 93/05064 und deckt z. B. alle Verbindungen ab, in denen die primäre Sulfamatfunktion an einem Sechsring gebunden ist. In der 2-Stellung des Steroidgerüstes spezifisch substituierte Verbindungen sind wiederum nicht explizit offenbart.

WO 96/05216 betrifft an C2-unsubstituierte Estra-1,3,5(10)-trien-Sulfamat-Derivate.

WO 96/05217 betrifft pharmazeutische Zusammensetzungen enthaltend Wirkstoffe der allgemeinen Formel 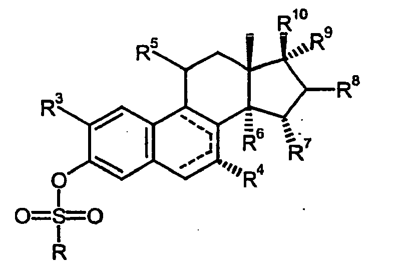 worin R = NH₂; R³ = C₁₋₅-Alkoxygruppe, OH; R⁸, R⁹ und R¹⁰ voneinander unabhängig = H, OH; R⁹ und R¹⁰ zusammen = O die Bedeutung haben können. Die darin offenbarten pharmazeutischen Zusammensetzungen können zur weiblichen Fertilitätskontrolle; klimakterischen HRT und zur Behandlung gynäkologischer und andrologischer Krankheitsbilder, wie Mamma- bzw. Prostatakarzinom verwendet werden.

WO 97/14712 betrifft Steroidsulfamat-Derivate der allgemeinen Formel 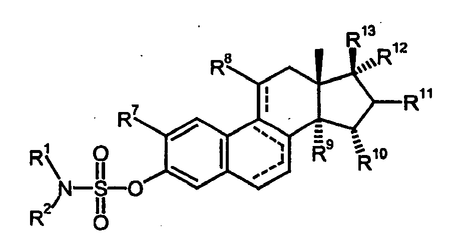 worin R¹ eine Acyl-, Alkoxycarbonyl-, Aminocarbonyl-, Sulfonyl- oder Sulfonamidylgruppe; R² ein Wasserstoff- oder ein Metallatom; R⁷ und R⁸ unabhängig voneinander H, OH und C₁₋₅-Alkoxy; R¹³, R¹², R¹¹ unabhängig voneinander H oder OH darstellen können.

WO 98/42729 betrifft 16-Halogensubstituierte-1,3,5-estratriene-3-monosulfamate sowie 3,17β-Bissulfamate, die an C2 alkoxysubstituiert sein können. Die 16-Halogensubstitution erhöht sowohl die Sulfatase-Hemmwirkung als auch die Estrogenität der entsprechenden Sulfamat-Derivate.

Die Einführung einer zu der 3-Sulfamat- zusätzlichen 17-Sulfamatfunktion setzt die Estrogenität drastisch herab.

WO 98/24802 betrifft Sulfamate, die die Estronsulfatase hemmen. 2-Methoxyestronsulfamat wird explizit genannt. Als potentielles Therapiegebiet findet Mammakarzinom, nicht jedoch Prostatakarzinom in der Beschreibung Erwähnung.

Auch WO 99/33858 beschreibt Estronsulfatase-Inhibitoren der Formel 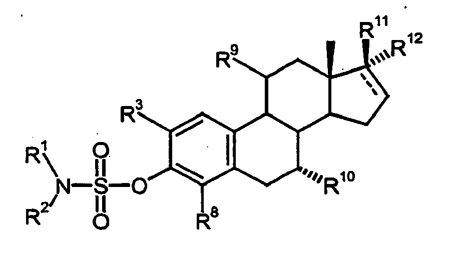 worin R¹ und R² unabhängig voneinander H, Alkyl, oder zusammen Piperidin-, Morpholin, Piperazin; R³ = H, CN, NO₂, CO₂R⁴; R⁸ = H, NO₂, NR⁶R⁷ darstellen. In der Beschreibung ist als mögliches Therapiegebiet Mammakarzinom erwähnt.

In WO 99/33859 sowie in US°2002/0032180 werden anti-estrogene Verbindungen beschrieben, die zur Behandlung verschiedener, vor allem estrogen-abhängiger Erkrankungen geeignet sind. Bevorzugte Verbindungen weisen einen Estra-1,3,5(10)-trien-Grundkörper auf und sind an Position 11 und 17 substituiert. Besonders bevorzugt sind 17-Desoxy-estra-1,3,5(10)-triene. Unter die allgemeinen Formeln fallen auch 2-substituierte D-Homo-estra-1,3,5(10)-trien-3yl-sulfamate, jedoch werden entsprechende Verbindungen nicht explizit genannt.

WO 99/64013 betrifft eine pharmazeutische Zusammensetzung eines Sulfamat-Derivates mit einem Zellsignalmodifier (wie z.B. TNFα). 2-Methoxyestronsulfamat wird in dieser Kombination als bevorzugtes Sulfamat explizit beansprucht; es fallen aber zahlreiche weitere Steroid-3-sulfamate unter den Umfang der allgemeinen Formel. Als Wirkmechanismus für die erfindungsgemäßen pharmazeutischen Zusammensetzungen bzw. für die darin enthaltenen Steroid-3-sulfamate (bevorzugt mit mindestens einem 2-Alkoxysubstituenten) werden 1) Hemmung der Glucoseaufnahme in Tumorzellen, 2) Hemmung der Tumorangiogenese, 3) Abbau der Mikrotubuli; 4) Induzierung von Apoptose beschrieben. WO 00/76487 betrifft Stoffe, die die TNFα-induzierte Aromatase-Aktivität hemmen. Als solche werden 2-Alkoxyestron-3-sulfamate, bevorzugt 2-Methoxyestronsulfamat beansprucht.

WO 01/30803 betrifft B-Homöstra-1,3,5(10)triene als Modulatoren der Tubulinpolymerisation. Insbesondere offenbart sie Verbindungen der allgemeinen Formel 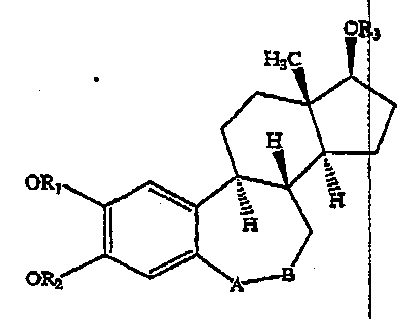 worin gemäß den bevorzugten Ausführungsformen A C=O, NR⁴; CHOH oder CHNHCOR⁶ und B CH₂ bedeuten, R¹ ausgewählt ist aus der Gruppe H, Methyl, Ethyl, I-Propenyl, n-Propyl und i-Propyl, R² und R⁴ ausgewählt sind aus der Gruppe H, Methyl, Ethyl, n-Propyl, i-Propyl, Acetyl, Propionyl, Butyryl und Isobutyryl, R³ ausgewählt ist aus der Gruppe H, Acetyl, Propionyl, Butyryl und Isobutyryl und R⁵ ausgewählt ist aus der Gruppe H, Acetyl, Propionyl, Butyryl und Isobutyryl.

WO 01/18028 beschreibt nicht-estrogene Estronsulfatase-inhibierende *N*-Acyl-18a-substituierte Steroid-3-sulfamate, wie z.B. 16α-Fluor-2-methoxy-18a-homoestradiol-(*N*-acetylsulfamat) bzw. 16α-Fluor-2-methoxy-18a-homoestron-(*N*-acetylsulfamat).

In Cancer 2000, 85, 983-994 werden die 2-Methoxyestradiol-, Docetaxel- und Paclitaxel-induzierte Apoptose in Hepatomazellen und deren Korrelation mit reaktiven Sauerstoffspezien verglichen.

Potter et al. Int. J. Cancer 2000, 85, 584-589 untersuchen die Wirkung von 2-Methoxyestronsulfamat im Vergleich zu 2-Methoxyestron auf das Wachstum von Brustkrebszellen und induzierte Mammatumoren und finden, dass 2-Methoxyestronsulfamat ein beachtliches therapeutisches Potential zur Behandlung von Brustkrebs aufweist.

Potter et. al. Molecular Cellular Endocrinology 2000, 160, 61-66 untersuchen die Hemmung der Deoxyglucoseaufnahme in MCF-7-Brustkrebszellen durch 2-Methoxyestron und 2-Methoxyestron-3-sulfamat, die die Glucoseaufnahme um 25 bis 49% bei 10µM (ebenso 2-Methoxyestradiol und 2-Methoxyestron) hemmen, und folgern, dass die Verbindungen durch ihr Vermögen die Glucoseaufnahme zu hemmen, therapeutisches Potential zur Hemmung von Brustkrebs haben könnten.

Potter et. al. Cancer Research 2000, 60, 5441-5450 beschreibt 2-Methoxyestronsulfamat und 2-Ethoxyestronsulfamat als neue antimikrotubullenaktive Verbindungen, die *in vitro* Antikrebsaktivität in Mammakarzinomzellen aufwiesen, und daher auch eventuell *in vivo* aktiv sein könnten. In J. Steroid Biochem. Mol. Biol. 1999, 69, 227-238 wird berichtet, dass die Hemmung der Steroidsulfatase-Aktivität ein wichtiger Ansatzpunkt bei der Behandlung von hormonabhängigen Mammakarzinomen ist. Explizit werden 2-Methoxyestronsulfamat, 17-Deoxyestronsulfamat und Estronsulfamat aufgeführt. Mono- bzw. bicyclische, nicht steroidale Sulfamate hemmen zwar die Steroidsulfatase, jedoch nicht so effektiv wie die entsprechenden Steroidderivate.

Die Aufgabe der vorliegenden Erfindung besteht darin, weitere Verbindungen zur Verfügung zu stellen, die die Tubulinpolymerisation wirksam hemmen.

Die Aufgabe der vorliegenden Erfindung wird erfindungsgemäß durch die Bereitstellung von 2-substituierten D-Homoestra-1,3,5(10)-trien-3-yl-sulfamaten der allgemeinen Formel I gelöst: 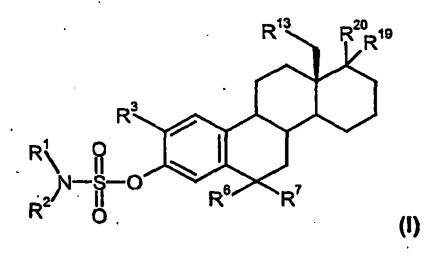 worin
- R¹ und R²: unabhängig voneinander ein Wasserstoffatom, eine Methyl-, C₁-C₄-Acyl- oder eine Benzoylgruppe,
- R³: eine C₁-C₅-Alkyl, eine C₁-C₅-Alkyloxygruppe oder einen Rest -O-CₙFₘHₒ, wobei n=1,2,3,4,5 oder 6, m>1 und m+o=2n+1 ist,
- R⁶ und R⁷: unabhängig voneinander ein Wasserstoffatom, eine Hydroxy-, Amino-oder NHR⁸-Gruppe, wobei
R⁸ eine Acetylgruppe ist,
- oder R⁶ und R⁷: zusammen ein Oxim NOH,
- R¹³: ein Wasserstoffatom oder eine Methylgruppe,
- R¹⁹: ein Wasserstoff- oder Fluoratom,
- R²⁰: ein Wasserstoff-oder Fluoratom oder eine Hydroxy- bzw. C₁-C₅-Alkyloxybzw. C₁-C₅-Alkylgruppe oder einen Rest -CₙFₘHₒ wobei n=1,2,3,4,5 oder 6, m>1 und m+o=2n+1 oder eine Gruppe -OSO₂NR¹R²
- R¹⁹ und R²⁰: zusammen ein Sauerstoffatom, eine Methylen-, Difluormethylen- oder Monofluormethylengruppe oder ein Oxim NOR²¹, wobei
R²¹ ein Wasserstoffatom oder eine C₁-C₅-Alkylgruppe ist,
bedeuten,
sowie ihre pharmazeutisch verträglichen Salze.

Gemäß einer zweiten Ausführungsform betrifft die Erfindung 2-Substituierte D-Homöstra-1,3,5(10)-trien-3-yl-sulfamate, wie sie oben definiert wurden, zur Verwendung als Medikament.

Gemäß einer dritten Ausführungsform betrifft die Erfindung 2-Substituierte D-Homöstra-1,3,5(10)-trien-3-yl-sulfamate, wie sie oben definiert wurden, für die Herstellung eines Medikaments zur Behandlung von Tumorerkrankungen.

Gemäß einer vierten Ausführungsform betrifft die Erfindung 2-Substituierte D-Homöstra-1,3,5(10)-trien-3-yl-sulfamate, wie sie oben definiert wurden, für die Herstellung eines Medikaments zur Behandlung von Brustkrebs.

Gemäß einer fünften Ausführungsform betrifft die Erfindung 2-Substituierte D-Homöstra-1,3,5(10)-trien-3-yl-sulfamate, wie sie oben definiert wurden, für die Herstellung eines Medikaments zur Behandlung von Prostatakrebs.

Gemäß einer fünften Ausführungsform betrifft die Erfindung 2-Substituierte D-Homöstra-1,3,5(10)-trien-3-yl-Sulfamate, wie sie oben definiert wurden, für die Herstellung eines Medikaments zur Behandlung von Brustkrebs.

Gemäß einer sechsten Ausführungsform betrifft die Erfindung pharmazeutische Zubereitungen, die wenigstens eine Verbindung der allgemeinen Formel I, wie sie oben definiert wurde, zusammen mit pharmazeutisch verträglichen Adjuvantien und/oder Vehikelflüssigkeiten enthalten.

Weiterhin umfasst die vorliegende Erfindung die neuen Verbindungen als pharmazeutisch Wirkstoffe, ihre therapeutische Anwendung und die pharmazeutischen Darreichungsformen, die die neuen Substanzen enthaltende.

Die erfindungsgemäßen Verbindungen der allgemeinen Formel (I) oder deren pharmazeutisch annehmbare Salze können für die Herstellung eines Arzneimittals, insbesondere zur Behandlung Tumorerkrankungen die sich durch die Hemmung der Tubulinpolymerisation positiv beeinflussen lassen, eingesetzt werden.

Es wurde festgestellt, dass die erfindungsgemäßen 2-Substituierten D-Homöstra-1,3,5(10)-trien-3-yl-sulfamate in vitro die Tubulinpolymerisation überraschend stärker hemmen als 2-Methoxyöstradiol. Die erfindungsgemäßen Verbindungen hemme die Proliferation von Tumorzellen und zeigen auch in vivo Antitumorwirkung.

Weiterhin verfügen die erfindungsgemäßen Verbindungen über eine bessere orale Bioverfügbarkeit als 2-Methoxyöstradiol.

Bei den C₁₋₆-Alkylgruppen für R³ oder R²⁰ kann es sich durchweg um eine Methyl-, Ethyl-, n-Propyl-, iso-Propyl-, n-, iso-, oder tert.-Butyl-, n-, iso- oder neo-Pentylgruppe handeln.

Für einen Acylrest R¹ und R² kann ein Formyl-, Acetyl-, Propionyl-, Butyryl- oder iso-Butyrylrest stehen.

Für den C₁-C₆-Alkoxyrest R³ oder R²⁰ kann eine Methoxy-, Ethoxy-, n-Propoxy-, iso-Propoxy-, n-, iso-, oder tert.-Butoxy, n-, iso-, oder neo-Pentoxygruppe stehen.

Bevorzugt gemäß vorliegender Erfindung sind Verbindungen der allgemeinen Formel I, in denen:
- R¹: H, Methyl, Acetyl, Propionyl, Butyryl, insbesondere H
- R²: H, Acyl
- R³: Methyl, Ethyl, Methoxy, Ethoxy, 2,2,2-Trifluorethoxy
- R⁶ und R⁷: beide Wasserstoff oder zusammen Oxim
- R¹³: H oder Methyl
- R¹⁹: H
- R²⁰: H, OH, C₁-C₅-Alkyloxy darstellen, insbesondere H, OH
ist.

Die nachstehend genannten Verbindungen sowie deren Verwendung sind erfindungsgemäß besonders bevorzugt:
1) 2-Methoxy-17a-oxo-17a-homoestra-1,3,5(10)-trien-3-yl sulfamat (1)
2) 2-Methoxy-17a-oxo-17a-homoestra-1,3,5(10)-trien-3-yl (N-acetyl)-sulfamat
3) 2-Methoxy-17a-homoestra-1,3,5(10)-trien-3-yl sulfamat (2)
4) 2-Methoxy-17a-homoestra-1,3,5(10)-trien-3-yl (N-acetyl)-sulfamat
5) 2-Methoxy-6-oximino-17a-homoestra-1,3,5(10)-trien-3-yl sulfamat
6) 2-Methoxy-6-(*O*-methyloximino)-17a-homoestra-1,3,5(10)-trien-3-yl sulfamat
7) 6α-Hydroxy-2-methoxy-17a-homoestra-1,3,5(10)-trien-3-yl sulfamat
8) 6α-Acetylamino-2-methoxy-17a-homoestra-1,3,5(10)-trien-3-yl sulfamat
9) 2-Methoxy-6-oxo-17a-homoestra-1,3,5(10)-trien-3-yl sulfamat
10) 17aα-Hydroxy-2-methoxy-17a-homoestra-1,3,5(10)-trien-3-yl sulfamat (3a)
11) 17aβ-Hydroxy-2-methoxy-17a-homoestra-1,3,5(10)-trien-3-yl sulfamat (3b)
12) 2-Methoxy-17a-homoestra-1,3,5(10)-trien-3,17aβ-diyl bissulfamat (4)
13) 2-Methoxy-17a-homoestra-1,3,5(10)-trien-3,17aβ-diyl bis-(N-acetyl)-sulfamat
14) 17a-Difluor-2-methoxy-17a-homoestra-1,3,5(10)-trien-3-yl sulfamat
15) 17aα-Fluor-2-methoxy-17a-homoestra-1,3,5(10)-trien-3-yl sulfamat
16) 17aβ-Fluor-2-methoxy-17a-homoestra-1,3,5(10)-trien-3-yl sulfamat
17) 2-Methoxy-17a-oximino-17a-homoestra-1,3,5(10)-trien-3-yl sulfamat
18) 2-Methoxy-17a-(methyloximino)-17a-homoestra-1,3,5(10)-trien-3-yl sulfamat
19) 2,17aβ-Dimethoxy-17a-homoestra-1,3,5(10)-trien-3-yl sulfamat
20) 2,17aβ-Dimethoxy-17a-homoestra-1,3,5(10)-trien-3-yl (N-acetyl)-sulfamat
21) 17aβ-Ethoxy-2-methoxy-17a-homoestra-1,3,5(10)-trien-3-yl sulfamat
22) 17aβ-Ethoxy-2-methoxy-17a-homoestra-1,3,5(10)-trien-3-yl (N-acetyl)-sulfamat
23) 2-Methoxy-17aβ-(n-propoxy)-17a-homoestra-1,3,5(10)-trien-3-yl sulfamat
24) 2-Methoxy-17aβ-methyl-17a-homoestra-1,3,5(10)-trien-3-yl sulfamat
25) 17aβ-Difluormethyl-2-methoxy-17a-homoestra-1,3,5(10)-trien-3-yl sulfamat
26) 17aβ-Fluormethyl-2-methoxy-17a-homoestra-1,3,5(10)-trien-3-yl sulfamat
27) 17aβ-Ethyl-2-methoxy-17a-homoestra-1,3,5(10)-trien-3-yl sulfamat
28) 2-Methoxy-17a(20)-methylen-17a-homoestra-1,3,5(10)-trien-3-yl sulfamat
29) 17a(20)-Difluormethylen-2-methoxy-17a-homoestra-1,3,5(10)-trien-3-yl sulfamat
30) 17a(20)-Fluormethylen-2-methoxy-17a-homoestra-1,3,5(10)-trien-3-yl sulfamat
31) 2-Methoxy-17a-oxo-17a,18a-dihomoestra-1,3,5(10)-trien-3-yl sulfamat
32) 2-Methoxy-17a-oxo-17a,18a-dihomoestra-1,3,5(10)-trien-3-yl (N-acetyl)-sulfamat
33) 2-Methoxy-17a,18a-dihomoestra-1,3,5(10)-trien-3-yl sulfamat
34) 2-Methoxy-17a,18a-dihomoestra-1,3,5(10)-trien-3-yl (N-acetyl)-sulfamat
35) 2-Methoxy-6-oximino-17a,18a-dihomoestra-1,3,5(10)-trien-3-yl sulfamat
36) 2-Methoxy-6-(*O*-methyloximino)-17a,18a-dihomoestra-1,3,5(10)-trien-3-yl sulfamat
37) 6α-Hydroxy-2-methoxy-17a,18a-dihomoestra-1,3,5(10)-trien-3-yl sulfamat
38) 6α-Acetylamino-2-methoxy-17a,18a-dihomoestra-1,3,5(10)-trien-3-yl sulfamat
39) 17aβ-Hydroxy-2-methoxy-17a,18a-dihomoestra-1,3,5(10)-trien-3-yl sulfamat
40) 2-Methoxy-17a,18a-dihomoestra-1,3,5(10)-trien-3,17aβ-diyl bissulfamat
41) 2-Methoxy-17a,18a-dihomoestra-1,3,5(10)-trien-3,17aβ-diyl bis-(N-acetyl)-sulfamat
42) 17a-Difluor-2-methoxy-17a,18a-dihomoestra-1,3,5(10)-trien-3-yl sulfamat
43) 17aα-Fluor-2-methoxy-17a,18a-dihomoestra-1,3,5(10)-trien-3-yl sulfamat
44) 17aβ-Fluor-2-methoxy-17a,18a-dihomoestra-1,3,5(10)-trien-3-yl sulfamat
45) 2-Methoxy-17a-oximino-17a,18a-dihomoestra-1,3,5(10)-trien-3-yl sulfamat
46) 2-Methoxy-17a-(methyloximino)-17a,18a-dihomoestra-1,3,5(10)-trien-3-yl sulfamat
47) 2,17aβ-Dimethoxy-17a,18a-dihomoestra-1,3,5(10)-trien-3-yl sulfamat
48) 17aβ-Ethoxy-2-methoxy-17a,18a-dihomoestra-1,3,5(10)-trien-3-yl sulfamat
49) 2-Methoxy-17aβ-(n-propoxy)-17a,18a-dihomoestra-1,3,5(10)-trien-3-yl sulfamat
50) 2-Methoxy-17aβ-methyl-17a,18a-dihomoestra-1,3,5(10)-trien-3-yl sulfamat
51) 17aβ-Difluormethyl-2-methoxy-17a,18a-dihomoestra-1,3,5(10)-trien-3-yl sulfamat
52) 17aβ-Fluormethyl-2-methoxy-17a,18a-dihomoestra-1,3,5(10)-trien-3-yl sulfamat
53) 17aβ-Ethyl-2-methoxy-17a,18a-dihomoestra-1,3,5(10)-trien-yl sulfamat
54) 2-Methoxy-17a(20)-methylen-17a,18a-dihomoestra-1,3,5(10)-trien-3-yl sulfamat
55) 17a(20)-Difluormethylen-2-methoxy-17a,18a-dihomoestra-1,3,5(10)-trien-3-yl sulfamat
56) 17a(20)-Fluormethylen-2-methoxy-17a,18a-dihomoestra-1,3,5(10)-trien-3-yl sulfamat
57) 2-Ethyl-17a-oxo-17a-homoestra-1,3,5(10)-trien-3-yl sulfamat
58) 2-Ethyl-17a-oxo-17a-homoestra-1,3,5(10)-trien-3-yl (N-acetyl)-sulfamat
59) 2-Ethyl-17a-homoestra-1,3,5(10)-trien-3-yl sulfamat
60) 2-Ethyl-17a-homoestra-1,3,5(10)-trien-3-yl (N-acetyl)-sulfamat
61) 2-Ethyl-17aβ-hydroxy-17a-homoestra-1,3,5(10)-trien-3-yl sulfamat
62) 2-Ethyl-17a-homoestra-1,3,5(10)-trien-3,17aβ-diyl bissulfamat
63) 2-Ethyl-17a-homoestra-1,3,5(10)-trien-3,17aβ-diyl bis-(N-acetyl)-sulfamat
64) 2-Ethyl-17aβ-methoxy-17a-homoestra-1,3,5(10)-trien-3-yl sulfamat
65) 2-Ethyl-17aβ-ethoxy-17a-homoestra-1,3,5(10)-trien-3-yl sulfamat

### Pharmakologische Daten

### 1. Hemmung der Tubulinpolymerisation

Die erfindungsgemäßen Verbindungen wurden in verschiedenen Modellen getestet. Die erfindungsgemäßen Verbindungen der allgemeinen Formel I zeichnen sich dadurch aus, dass sie die Tubulinpolymerisation stärker hemmen als 2-Methoxyestradiol. Die *in vitro* Testung der Tubulinpolymerisationsbeeinflussung wurde folgendermaßen durchgeführt:

Mikrotubuläres Protein wurde nach Shelanski et. al. (Shelanski et. al. Proc. Natl. Acad. Sci. USA 1973, 70, 765-8) über zyklische Assemblierung/ Deassemblierung aus Schweinehirn gereinigt. Das verwendete Puffersystem hatte folgende Zusammensetzung: 20 mM PIPES (1,4-Piperazine-diethane-sulfonsäure, pKa 6,8), 80 mM NaCl, 0,5 mM MgCl₂, 1 mM EGTA [Ethylenglykol-bis-(2-aminoethylen)-tetraessigsäure].

Für die Wirkstofftestung wurden Proteinkonzentrationen von 1 mg/ ml (ca. 10⁻⁵ mM Tubulin) eingesetzt. Die Proteinbestimmung erfolgte nach der Lowry-Methode (Lowry et al. J. Bio!. Chem. 1951, 193, 265-75) mit Rinderserumalbumin als Standard. Die Assemblierung von Mikrotubuli erfolgte in Gegenwart von 0.25 mM GTP und Erwärmen der Proben auf 37°C.

Die Mikrotubulusbildung wurde mit Hilfe der Turbidimetrie bei einer Wellenlänge von 340 nm geprüft. Der Gleichgewichtszustand, bei dem das mikrotubuläre Protein keinen Zuwachs der Assemblatkonzentration (entsprechend der Mikrotubuluskonzentration) und der Trübungswert keinen Anstieg mehr aufweist, wird typischerweise nach 20 Minuten erreicht.

Die Testung der Wirkstoffe erfolgte durch deren Zugabe am Anfang der Assemblierung oder im Gleichgewichtszustand. Abweichungen der Trübungskurven von der Kontrolle charakterisieren ihre Wirksamkeit. Zur Wirkungskontrolle und Bewertung der Trübungsmesswerte wurde stets eine transmissionselektronenmikroskopische Untersuchung (CEM 902 A, Zeiss/ Oberkochen) der Assemblate nach Negativfärbung mit 1 %igem wässrigen Uranylazetat durchgeführt.

**Tab. 1.**

| **Verbindung** | **Hemmung der Tubulin-polymerisation** **IC₅₀ [µM]** |
|---|---|
| 2-Methoxyestradiol | 2,7 |
| **(2)** | 0,95 |

### 2. Hemmung der Zellproliferation

Die erfindungsgemäßen Verbindungen zeichnen sich durch eine potente Hemmung der Zellproliferation aus.

Zellkulturen der folgenden Zelllinien wurden in 96well Mikrotiterplatten angelegt:
1. MaTu/ADR Multidrug-resistente humanen Brusttumorzellen (Epo GmbH Berlin), 5000 Zellen/Well.
2. HCT116 humane Kolontumorzellen (ATCC CCL-247), 3000 Zellen/Well.
3. NCI-H460 humane nicht-kleinzelliges Lungenkarzinomzellen (ATCC HTB-177), 3000 Zellen/Well.
4. DU145 humane Prostatatumorzellen (ATCC HTB-81), 5000 Zellen/Well.
5. HMVEC humane primäre dermale mikrovaskuläre Endothelzellen, 7500 Zellen/Well.

Nach 24 Stunden Inkubationszeit in einem Zellkulturbrutschrank bei 37°C wurden die Zellen einer Mikrotiterplatte mit Kristallviolett gefärbt (Referenzplatte), während die Zellen in den Testplatten für 4 Tage mit den Testsubstanzen in den Konzentrationen 0.1-10 µM, sowie mit dem Lösungsmittel DMSO allein (Lösungsmittelkontrolle), inkubiert wurden. Die Zellproliferation wurde durch Färbung der Zellen mit Kristallviolett bestimmt. Die Extinktion des Kristallvioletts wurde photometrisch bei 595 nm bestimmt. Die Prozentzahl der Änderung der Zellzahl in den Testplatten wurde nach Normalisation der Extinktionswerte auf die Referenzplatte (0%) und auf die Lösungsmittelkontrollen (100%) bestimmt. Die halbmaximale Inhibition des Zellwachstums (IC50) wurde als die Substanzkonzentration bestimmt, bei der 50% der Zellzahl der Lösungsmittelkontrollen vorhanden waren.

| Verbindung | Hemmung der Zellproliferation IC50 [µM] | | | | |
|---|---|---|---|---|---|
| | NCI-H460 | HCT116 | DU145 | MaTu/ADR | HMVEC |
| **Taxol** | 0,004 | 0,004 | 0,004 | 0,4 | 0,004 |
| **2-Methoxyestradiol** | 1.8 | 1.1 | 1.9 | 0.2 | 2.2 |
| (1) | 0.18 | 0.18 | 0.5 | <0.1 | 0.22 |
| (2) | 0.6 | 0.6 | 0.6 | 0.2 | 0.5 |
| (4) | 1.8 | 1.8 | 2.8 | 0.8 | 0.6 |

### Dosierung

Im allgemeinen sind zufriedenstellende Resultate zu erwarten, wenn die täglichen Dosen einen Bereich von 5 µg bis 50 mg der erfindungsgemäßen Verbindung pro kg Körpergewicht umfassen. Bei größeren Säugetieren, beispielsweise dem Menschen, liegt eine empfohlene tägliche Dosis im Bereich von 10 µg bis 30 mg pro kg Körpergewicht.

Geeignete Dosierungen für die erfindungsgemäßen Verbindungen betragen von 0,005 bis 50 mg pro Tag pro kg Körpergewicht, je nach Alter und Konstitution des Patienten, wobei die notwendige Tagesdosis durch Einmal- oder Mehrfachabgabe appliziert werden kann.

Aufgrund der besonderen Depotwirkung der Estrogen-Sulfamate können die erfindungsgemäßen Verbindungen aber auch in größeren Abständen als einmal am Tag verabreicht werden.

Die Formulierung der pharmazeutischen Präparate auf Basis der neuen Verbindungen erfolgt in an sich bekannter Weise, indem man den Wirkstoff mit den in der Galenik gebräuchlichen Trägersubstanzen, Füllstoffen, Zerfallsbeeinflussern, Bindemitteln, Feuchthaltemitteln, Gleitmitteln, Absorptionsmitteln, Verdünnungsmitteln, Geschmackskorrigentien, Färbemitteln usw. verarbeitet und in die gewünschte Applikationsform überführt. Dabei ist auf Remington's Pharmaceutical Science, 15th ed. Mack Publishing Company, East Pennsylvania (1980) hinzuweisen.

Für die orale Applikation kommen insbesondere Tabletten, Dragees, Kapseln, Pillen, Pulver, Granulate, Pastillen, Suspensionen, Emulsionen oder Lösungen in Frage.

Für die parenterale Applikation sind Injektion- und Infusionszubereitungen möglich.

Für die intraartikulären Injektion können entsprechend zubereitete Kristallsuspensionen verwendet werden.

Für die intramuskuläre Injektion können wässrige und ölige Injektionslösungen oder Suspensionen und entsprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen als auch zur lokalen Therapie verwendet werden.

Zur pulmonalen Applikation der neuen Verbindungen können diese in Form von Aerosolen und Inhalaten verwendet werden.

Für die topische Auftragung sind Formulierungen in Gele, Salben, Fettsalben, Cremes, Pasten, Puder, Milch und Tinkturen möglich. Die Dosierung der Verbindungen der allgemeinen Formel I sollte in diesen Zubereitungen 0,01% - 20% betragen, um eine ausreichende pharmakologische Wirkung zu erzielen.

Die vorliegende Erfindung umfasst die Verbindungen der allgemeinen Formel I und deren Verwendung zur Herstellung eines Arzneimittels, insbesondere zur Behandlung von Tumorerkrankungen, die sich durch die Hemmung der Tubulinpolymerisation positiv beeinflussen lassen.

Die erfindungsgemäßen Verbindungen der allgemeinen Formel I werden bevorzugt zur Herstellung eines Arzneimittels zur Behandlung von Tumorerkrankungen der männlichen und weiblichen Keimdrüsen, männlichen und weiblichen Sexualorgane einschließlich der Brustdrüsen, insbesondere von Prostatakarzinomen oder Mammakarzinom verwendet.

Ferner betrifft die vorliegende Erfindung pharmazeutische Zusammensetzungen, die mindestens eine erfindungsgemäß besonders bevorzugte Verbindung, gegebenenfalls in Form eines pharmazeutisch/ pharmakologisch verträglichen Salzes, ohne oder zusammen mit pharmazeutisch verträglichen Hilfs- und/oder Trägerstoffen enthalten.

Diese pharmazeutischen Zusammensetzungen und Arzneimittel können zur oralen, rektalen, vaginalen, subkutanen, perkutanen, intravenösen oder intramuskulären Applikation vorgesehen sein. Sie enthalten neben üblichen Träger- und/ oder Verdünnungsmitteln mindestens eine erfindungsgemäß besonders bevorzugte Verbindung.

Die Arzneimittel der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder auch Depotformen.

Die pharmazeutischen Zusammensetzungen, die mindestens eine der erfindungsgemäßen Verbindungen enthalten, werden bevorzugt oral appliziert.

Es kommen auch parenterale Zubereitungen wie Injektionslösungen in Betracht. Weiterhin seien als Zubereitungen beispielsweise auch Suppositorien und Mittel zur vaginalen Anwendung genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylactat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titanoxid-oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Verbindungen der allgemeinen Formel I können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten.

Die Verbindungen der allgemeinen Formel I enthaltende Kapseln können beilspielsweise hergestellt werden, indem man die Verbindung(en) der allgemeinen Formel I mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyethylenglykol bzw. deren Derivaten herstellen.

Die erfindungsgemäßen Verbindungen können zur Therapie von Prostatakarzinomen mit einem oder mehreren der folgenden Wirkstoffe kombiniert verabreicht werden:
1) Antiandrogene wie CPA, Flutamid, Casodex etc.
2) Gonadrotrophormon (GnRH) Agonisten
3) 5α-Reduktase Hemmer wie Finasterid
4) Zytostatika
5) VEGF-Kinase-Inhibitoren
6) Antigestagene
7) Antiestrogene
8) Antisense Oligonukleotide
9) EGF-Antikörper
10)Estrogene

Darüber hinaus können die erfindungsgemäßen Verbindungen der allgemeinen Formel I zur Therapie und Prophylaxe weiterer oben nicht genannter Krankheitszustände eingesetzt werden.

Die erfindungsgemäßen Verbindungen der allgemeinen Formel I lassen sich wie nachstehend beschrieben herstellen:

Die Funktionalisierung des C-Atoms 2 eines Estra-1,3,5(10)-trien-17-on-derivates erfolgt vorzugsweise durch Friedel-Crafts-Acylierung wie in der Literatur beschrieben (T. Nambara et al. Chem. Pharm. Bull. 1979, 18, 474-480).

Nach Wechsel der Schutzgruppe in Position 3 wird durch Baeyer-Villiger-Oxidation ein 2-Carboxy-estra-1,3,5(10)-trien-17-on generiert (M.B. Smith, J. March, March's Advanced Organic Chemistry, 5. Edition, Wiley Sons 2001, 1417-1418 und dort zit. Lit.). Der Ester wird verseift und mit dem entsprechenden Alkylhalogenid unter basischen Bedingungen in einen 2-Alkylether überführt. Alternativ kann nun das 17-Keton wie bekannt reduziert und verethert werden. Die Spaltung der Schutzgruppe in Position 3 erfolgt wie in der Literatur beschrieben (T.W. Greene, P.G.M. Wuts, Protective Groups in Organic Synthesis, Wiley & Sons, 1999, 249-275). Dieses Verfahren oder andere aus der Literatur bekannte (P.N. Rao, J.W. Cessac, Steroids 2002, 67, 1065-1070 und dort zit. Lit.) sind entsprechend auf die 17a-Homo- bzw. 17a, 18a-Dihomoderivate anwendbar.

Die vorzugsweise durch Friedel-Crafts-Acylierung erhaltenen 2-Acylderivate können durch Reduktion mit Natriumborhydrid und anschließender Hydrierung in die entsprechenden 2-Alkylderivate übergeführt werden.

Ebenso können aus den 2-funktionalisierten Derivaten die entsprechenden 17a-Oxim-, 17a-Alkylen- (sog. Wittig-Reaktion, siehe z.B S. Schwarz et al. Pharmazie 2001, 56, 843-849), 17a-Difluormethylen-(Wadsworth-Emmons-Reaktion, S.R. Piettre, L. Cabanas, Tetrahedron Lett. 1996, 37, 5881-4884), 17α,β-Alkylderivate hergestellt (z.B. R.H. Peters et al., J. Med. Chem. 1989, 32, 1642; G.E. Agoston et al. WO02/42319) und anschließend in 3-Position sulfamoyliert werden.

Nach Cushman et al. (J. Med. Chem. 1997, 40, 2323) erfolgt die Synthese 6-funktionalisierter Estrogenderivate durch Oxidation des Acetyl-geschützen Estrogenderivates mit Chromtrioxid.

Ausgehend von den 2-funktionalisierten 17-Keto-Derivaten können 17-Oxirane (M. Hübner, I. Noack, J. prakt. Chem. 1972, 314, 667), und daraus die entsprechenden 17a-Homo-Derivate (M. Hübner, K. Ponsold, Z. Chem. 1982, 22, 186) hergestellt werden.

17a-Fluorierte Derivate können aus den etnsprechenden 17a-Oxo bzw. 17a-Hydroxy-Derivaten mit Diethylamino-schwefeltrifluorid hergestellt (M. Hudlicky, Org. Reactions 1988, 35, 513; J.T. Welch, Fluorine in Bioorganic Chemistry 1991, John Wiley, New York; S. Rozen et al. Tetrahedron Lett. 1979, 20, 1823-1826) und anschließend sulfamoyliert werden.

Die vorliegende Erfindung wird anhand der nachfolgenden Beispiele näher erläutert, ohne sie darauf einzuschränken:

### Herstellungsverfahren

### Allgemeine Vorschrift 1 zur Herstellung von 17a-Homoestra-1,3,5(10)-trien-3-ylsulfamaten

Es werden ein Äquivalent eines 17a-Homoestra-1,3,5(10)-trien-Derivates in Methylenchlorid unter Rühren gelöst bzw. suspendiert und mit 5 Äquivalenten 2,6-Di-tert.-butylpyridin versetzt. Anschließend werden unter Argon 10 Äquivalente Sulfamoylchlorid zugegeben und bei Raumtemperatur gerührt. Die Lösung wird bis zum vollständigen Umsatz gerührt (DC-Kontrolle, 1-5h) und dann mit Wasser versetzt. Bei säureempfindlichen Verbindungen wird vorher mit rund 10 Äquivalenten Triethylamin gepuffert. Die wässrige Phase wird mehrmals mit Dichlormethan oder Essigester extrahiert. Die vereinigten organischen Phasen werden über Natriumsulfat getrocknet und am Vakuum eingeengt und anschließend flashchromatographisch gereinigt.

### Allgemeine Vorschrift 2 zur Acylierung von Sulfamaten

Ein Äquivalent des 17a-Homoestra-1,3,5(10)-trien-Sulfamates bzw. Bisulfamates werden im Pyridin gelöst und unter Eiskühlung (0 bis 5°C) mit 5 Äquivalenten Anhydrid versetzt. Es wird 1h bei Raumtemperatur weitergerührt und dann mit Wasser versetzt. Die wässrige Phase wird mehrmals mit Dichlormethan oder Essigester extrahiert. Die vereinigten organischen Phasen werden mit 6N Salzsäure und anschließend mit Wasser und Natriumchloridlösung gewaschen. Danach wird über Natriumsulfat getrocknet und am Vakuum eingeengt und anschließend flashchromatographisch gereinigt.

Folgende erfindungsgemäßen Verbindungen wurden nach den genannten Vorschriften hergestellt:

### Beispiel 1

### 2-Methoxy-17a-oxo-17a-homoestra-1,3,5(10)-trien-3-yl sulfamat (1):

3.61 g 17α-Azidomethyl-3,17β-dihydroxy-2-methoxy-estra-1,3,5(10)-trien und 7.5 g Natriumiodid wurden in 250 mL Acetonitril suspendiert und bei Raumtemperatur langsam mit 15 mL Trimethylsilylchlorid versetzt. Nach 4h wurden weitere 4 mL Trimethylsilylchlorid zugegeben und nach weiteren 2.5h wurde mit ges. Natriumthiosulfatlsg und Wasser versetzt und mit Dichlormethan extrahiert (3x). Die vereinigten organischen Phasen wurden mit wässr. Natriumbicarbonatlsg, gewaschen, getrocknet und am Rotationsverdampfer eingeengt. Flashchromatographie (Cyclohexan/Essigester = 10:1 → 7:1 → 5:1) lieferte 2.12 g (67%) 3-Hydroxy-2-methoxy-17a-oxo-17a-homoestra-1,3,5(10)-trien als farblose Kristalle.
¹H-NMR (CDCl₃): δ = 1.13 (s, 3H; 18-CH₃), 2.62-2.71 (m, 1 H; 17-H), 2.77 (dd, 2H; 6-CH₂), 3.86 (s, 3H; 2-OCH₃), 5.48 (s, 1 H; 3-OH), 6.63, 6.78 (2 s, 2H; 1-H, 4-H).

492 mg 3-Hydroxy-2-methoxy-17a-oxo-17a-homoestra-1,3,5(10)-trien wurden nach der allgemeinen Synthesevorschrift zum Produkt umgesetzt und anschließend durch Flashchromatographie (Cyclohexan/Essigester = 3:1 → 2:1) gereinigt. Es wurden 545 mg (89%) 2-Methoxy-17a-oxo-17a-homoestra-1,3,5(10)-trien-3-yl sulfamat **(1)** als farblose Kristalle erhalten.
¹H-NMR (CDCl₃): δ = 1.13 (s, 3H; 18-CH₃), 2.63-2.71 (m, 1 H; 17-H), 2.74-2.84 (m, 2H; 6-CH₂), 3.88 (s, 3H; 2-OCH₃), 5.00 (s, 2H; NH₂), 6.93, 7.04 (2 s, 2H; 1-H, 4-H).

### Beispiel 2

### 2-Methoxy-17a-homoestra-1,3,5(10)-trien-3-yl sulfamat (2):

600 mg 3-Hydroxy-2-methoxy-17a-oxo-17a-homoestra-1,3,5(10)-trien wurden in 20 mL Triethylenglycol gelöst und unter Argon mit 15 mL Hydrazin-monohydrat und 0.8 g Kaliumhydroxid versetzt. Anschließend wurde 2h auf 130°C und anschließend weitere 1.5h auf 200°C erhitzt. Nach Abkühlen auf Raumtemperatur wurde mit 6N Salzsäure angesäuert und mit Dichlormethan extrahiert (3x). Die vereinigten organischen Phasen wurden mit ges. Natriumbicarbonatlsg, gewaschen, getrocknet und am Rotationsverdampfer eingeengt. Flashchromatographie (Cyclohexan/Essigester = 100:1 → 50:1 → 20:1) lieferte 541 mg (94%) 3-Hydroxy-2-methoxy-17a-homoestra-1,3,5(10)-trien als farblose Kristalle.
¹H-NMR (CDCl₃): δ = 0.85 (s, 3H; 18-CH₃), 2.71-2.74 (m, 2H; 6-CH₂), 3.85 (s, 3H; 2-OCH₃), 5.41 (s; 1H; 3-OH), 6.62, 6.79 (2 s, 2H; 1-H, 4-H).

253 mg 3-Hydroxy-2-methoxy-17a-homoestra-1,3,5(10)-trien wurden nach der allgemeinen Synthesevorschrift 1 zum Produkt umgesetzt und anschließend durch Flashchromatographie (Toluol/Essigester = 20:1 → 10:1) gereinigt. Es wurden 217 mg (68%) 2-Methoxy-17a-homoestra-1,3,5(10)-trien-3-yl sulfamat **(2)** in Form farbloser Kristalle erhalten.
¹H-NMR (CDCl₃): δ = 0.85 (s, 3H; 18-CH₃), 2.67-2.82 (m, 2H; 6-CH₂), 3.86 (s, 3H; 2-OCH₃), 4.97 (s, 2H; NH₂), 6.93, 7.02 (2 s, 2H; 1-H, 4-H).

### Beispiel 3

### 17aa-Hydroxy-2-methoxy-17a-homoestra-1,3,5(10)-trien-3-yl sulfamat (3a) und 17aβ-Hydroxy-2-methoxy-17a-homoestra-1,3,5(10)-trien-3-yl sulfamat (3b):

298 mg 2-Methoxy-17a-oxo-17a-homoestra-1,3,5(10)-trien-3-yl sulfamat **(2)** wurden in 20 mL Methanol und 10 mL Tetrahydrofuran gelöst und unter Eiskühlung mit 115 mg Natriumborhydrid versetzt. Nach 2h wurde mit Aceton versetzt und am Rotationsverdampfer eingeengt. Der Rückstand wurde mit 6N Salzsäure angesäuert und mit Dichlormethan extrahiert (2x). Die vereinigten organischen Phasen wurden mit ges. Natriumbicarbonatlsg, gewaschen, getrocknet und am Rotationsverdampfer eingeengt. Flashchromatographie (n-Hexan/Essigester = 3:2 → 1:1) lieferte 35 mg (12%) des α-Epimers **3a** sowie 276 mg (92%) des β-Epimers **3b** als amorphe Feststoffe.
**3a:** ¹H-NMR (CDCl₃): δ = 0.86 (s, 3H; 18-CH₃), 3.42 (dd, ³*J_{eq}* = ³*Jₐₓ* = 2.7 Hz, 1 H; 17aβ-H), 3.86 (s, 3H; 2-OCH₃), 5.14 (s, 2H; NH₂), 6.92, 7.02 (2 s, 2H; 1-H, 4-H).
**3b:** ¹H-NMR (CDCl₃): δ = 0.84 (s, 3H; 18-CH₃), 3.25 (dd, ³*J*= 4.3 und 11.3 Hz, 1H; 17aα-H), 3.87 (s, 3H; 2-OCH₃), 5.07 (s, 2H; NH₂), 5.29 (s, 1H; OH), 6.93, 7.03 (2 s, 2H; 1-H, 4-H).

### Beispiel 4

### 2-Methoxy-17a-homoestra-1,3,5(10)-trien-3,17aβ-diyl bissulfamat (4):

62 mg 17aβ-Hydroxy-2-methoxy-17a-homoestra-1,3,5(10)-trien-3-yl sulfamat (3b) wurden nach der allgemeinen Synthesevorschrift 1 zum Produkt umgesetzt und anschließend durch Flashchromatographie (Toluol/Essigester = 3:1) gereinigt. Es wurden 55 mg (74%) 2-Methoxy-17a-homoestra-1,3,5(10)-trien-3,17aβ-diyl bissulfamat (**4**) als farbloses Öl erhalten, welches langsam kristallisierte.
¹H-NMR (DMSO-d₆): δ = 0.84 (s, 3H; 18-CH₃), 3.76 (s, 3H; 2-OCH₃), 4.06 (dd, ³*J*= 4.3 und 11.7 Hz, 1H; 17aα-H), 6.97, 7.00 (2 s, 2H; 1-H, 4-H), 7.37 (s, 2H; NH₂), 7.82 (s, 2H; NH₂).

## Patentansprüche

1. 2-Substituierte D-Homöstra-1,3,5(10)-trien-3-yl-sulfamate der allgemeinen Formel I 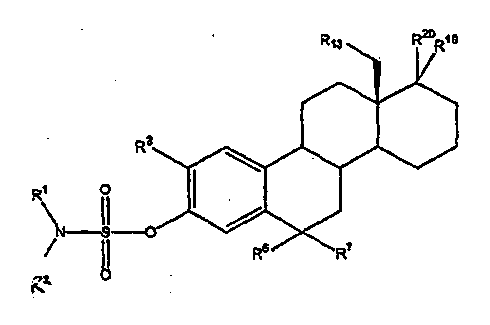 worin
R¹ und R² unabhängig voneinander ein H-Atom, eine Methyl-, eine C₁₋₄-Acyl- oder eine Benzoylgruppe,
R³ eine C₁₋₅-Alkyl- oder C₁₋₅-Alkoxygruppe oder einen Rest -O-CₙFₘHₒ bedeuten, wobei n = 1, 2, 3, 4, 5 oder 6, m > 1 und m+o=2n+1,
R⁶ und R⁷ unabhängig voneinander ein H-Atom, eine Hydroxy-, eine Amino- oder eine NHR⁸-Gruppe bedeuten, wobei
R⁸ eine Acetylgruppe ist, oder
R⁶ und R⁷ zusammen ein Oxim NOH sind,
R¹³ ein H-Atom oder eine Methylgruppe,
R¹⁹ ein H-Atom oder ein F-Atom und
R²⁰ ein H-Atom oder F-Atom oder eine Hydroxy-, eine C₁₋₅-Alkyloxy- oder eine C₁₋₅-Alkylgruppe oder einen Rest-CₙFₘHₒ, wobei n = 1, 2, 3, 4, 5 oder 6, m > 1 und m+o=2n+1, oder eine Gruppe OSO₂NR¹R²-bedeuten
R¹⁹ und R²⁰ zusammen ein O-Atom, eine Methylen-, eine Difluormethylen- oder eine Monofluormethylengruppe oder ein Oxim NOR²¹- bedeuten, wobei
R²¹ ein H-Atom oder eine C₁₋₅-Alkylgruppe ist, sowie ihre pharmazeutisch verträglichen Salze.

2. 2-Substituierte D-Homöstra-1,3,5(10)-trien-3-yl-sulfamate nach Anspruch 1, **dadurch gekennzeichnet, daß** R³ eine Methyl-, Ehtyl-, Methoxy-, Ethoxy- oder 2,2,2-Trifluorethoxygruppe darstellt.

3. 2-Substituierte D-Homöstra-1,3,5(10)-trien-3-yl-sulfamate nach Anspruch 2, worin R³ eine Methoxygruppe darstellt.

4. 2-Substituierte D-Homöstra-1,3,5(10)-trien-3-yl-sulfamate nach den Ansprüchen 1 bis 3, worin R⁶ und R⁷ in jedem Fall ein H-Atom darstellen.

5. 2-Substituierte D-Homöstra-1,3,5(10)-trien-3-yl-sulfamate nach den Ansprüchen 1 bis 4, worin R¹⁹ ein H-Atom darstellt.

6. 2-Substituierte D-Homöstra-1,3,5(10)-trien-3-yl-sulfamate nach den Ansprüchen 1 bis 5, worin R²⁰ ein H-Atom oder eine Hydroxygruppe darstellt.

7. 2-Substituierte D-Homöstra-1,3,5(10)-trien-3-yl-sulfamate nach den Ansprüchen 1 bis 6, worin R¹ ein H-Atom darstellt.

8. 2-Substituierte D-Homöstra-1,3,5(10)-trien-3-yl-sulfamate nach den Ansprüchen 1 bis 7, worin R² ein H-Atom oder eine Acylgruppe darstellt.

9. 2-Substituierte D-Homöstra-1,3,5(10)-trien-3-yl-sulfamate nach den Ansprüchen 1 bis 8, worin R¹³ ein H-Atom oder eine Methylgruppe darstellt.

10. 2-Substituierte D-Homöstra-1,3,5(10)-trien-3-yl-sulfamate nach Anspruch 1, und zwar
1) 2-Methoxy-17a-oxo-17a-homöstra-1,3,5(10)-trien-3-yl-sulfamat,
2) 2-Methoxy-17a-oxo-17a-homöstra-1,3,5(10)-trien-3-yl (N-Acetyl)-sulfamat,
3) 2-Methoxy-17a-homöstra-1,3,5(10)-trien-3-yl-sulfamat,
4) 2-Methoxy-17a-homöstra-1,3,5(10)-trien-3-yl (N-Acetyl)-sulfamat,
5) 2-Methoxy-6-oximino-17a-homöstra-1,3,5(10)-trien-3-yl-sulfamat,
6) 2-Methoxy-6-(O-methyloximino)-17a-homöstra-1,3,5(10)-trien-3-yl-sulfamat,
7) 6α-Hydroxy-2-methoxy-17a-homöstra-1,3,5(10)-trien-3-yl-sulfamat,
8) 6α-Acetylamino-2-methoxy-17a-homöstra-1,3,5(10)-trien-3-yl-sulfarnat,
9) 2-Methoxy-6-oxo-17a-homöstra-1,3,5(10)-trien-3-yl-sulfamat,
10) 17aα-Hydroxy-2-methoxy-17a-homöstra-1,3,5(10)-trien-3-yl-sulfamat,
11) 17aβ-Hydroxy-2-methoxy-17a-homöstra-1,3,5(10)-trien-3-yl-sulfamat,
12) 2-Methoxy-17a-homöstra-1,3,5(10)-trien-3,17aβ-diyl-bissulfamat,
13) 2-Methoxy-17a-homöstra-1,3,5(10)-trien-3,17aβ-diyl-bis-(N-acetyl)-sulfamat,
14) 17a-Difluor-2-methoxy-17a-homöstra-1,3,5(10)-trien-3-yl-sulfamat,
15) 17aα-Fluor-2-methoxy-17a-homöstra-1,3,5(10)-trien-3-yl-sulfamat,
16) 17aβ-Fluor-2-methoxy-17a-homöstra-1,3,5(10)-trien-3-yl-sulfamat,
17) 2-Methoxy-17a-oximino-17a-homöstra-1,3,5(10)-trien-3-yl-sulfamat,
18) 2-Methoxy-17a-(methyloximino)-17a-homöstra-1,3,5(10)-trien-3-yl-sulfamat,
19) 2,17aβ-Dimethoxy-17a-homöstra-1,3,5(10)-trien-3-yl-sulfamat,
20) 2,17aβ-Dimethoxy-17a-homöstra-1,3,5(10)-trien-3-yl(N-Acetyl)-sulfamat,
21) 17aβ-Ethoxy-2-methoxy-17a-homöstra-1,3,5(10)-trien-3-yl-sulfamat,
22) 17aβ-Ethoxy-2-methoxy-17a-homöstra-1,3,5(10)-trien-3-yl (N-Acetyl)-sulfamat,
23) 2-Methoxy-17aβ-(n-propoxy)-17a-homöstra-1,3,5(10)-trien-3-yl-sulfamat,
24) 2-Methoxy-17aβ-methyl-17a-homöstra-1,3,5(10)-trien-3-yl-sulfamat,
25) 17aβ-Difluormethyl-2-methoxy-17a-homöstra-1,3,5(10)-trien-3-yl-sulfamat,
26) 17aβ-Fluormethyl-2-methoxy-17a-homöstra-1,3,5(10)-trien-3-yl-sulfamat,
27) 17aβ**-**Ethyl-2-methoxy-17a-homöstra-1,3,5(10)-trien-3-yl-sulfamat,
28) 2-Methoxy-17a(20)-methylen-17a-homöstra-1,3,5(10)-trien-3-yl-sulfamat,
29) 17a(20)-Difluormethylen-2-methoxy-17a-homöstra-1,3,5(10)-trien-3-yl-sulfamat,
30) 17a(20)-Fluormethylen-2-methoxy-17a-homöstra-1,3,5(10)-trien-3-yl-sulfamat,
31) 2-Methoxy-17a-oxo-17a,18a-dihomöstra-1,3,5(10)-trien-3-yl-sulfamat,
32) 2-Methoxy-17a-oxo-17a,18a-dihomöstra-1,3,5(10)-trien-3-yl(N-Acetyl)-sulfamat,
33) 2-Methoxy-17a,18a-dihomöstra-1,3,5(10)-trien-3-yl-sulfamat,
34) 2-Methoxy-17a,18a-dihomöstra-1,3,5(10)-then-3-yl(N-Acetyl)-sulfamat,
35) 2-Methoxy-6-oximino-17a,18a-dihomöstra-1,3,5(10)-trien-3-yl-sulfamat,
36) 2-Methoxy-6-(O-methyloximino)-17a,18a-dihomöstra-1,3,5(10)-trien-3-yl-sulfamat,
37) 6α-Hydroxy-2-methoxy-17a,18a-dihomöstra-1,3,5(10)-trien-3-yl-sulfamat,
38) 6α-Acetylamino-2-methoxy-17a,18a-dihomöstra-1,3,5(10)-trien-3-yl-sulfamat,
39) 17aβ-Hydroxy-2-methoxy-17a,18a-dihomöstra-1,3,5(10)-trien-3-yl-sulfamat,
40) 2-Methoxy-17a,18a-dihomöstra-1,3,5(10)-trien-3,17aβ-diyl-bissulfamat,
41)2-Methoxy-17a,18a-dihomöstra-1,3,5(10)-trien-3,7aβ-diyl-bis-(N-acetyl)-sulfamat,
42) 17a-Difluor-2-methoxy-17a,18a-dihomöstra-1,3,5(10)-trien-3-yl-sulfamat,
43) 17aα-Fluor-2-methoxy-17a,18a-dihomöstra-1,3,5(10)-trien-3-yl-sulfamat,
44) 17aβ-Fluor-2-methoxy-17a,18a-dihomöstra-1,3,5(10)-trien-3-yl-sulfamat,
45) 2-Methoxy-17a-oximino-17a,18a-dihomöstra-1,3,5(10)-trien-3-yl-sulfamat,
46)2-Methoxy-17a-(methyloximino)-17a,18a-dihomöstra-1,3,5(10)-trien-3-yl-sulfamat,
47) 2,17aβ-Dimethoxy-17a,8a-dihomöstra-1,3,5(10)-trien-3-yl-sulfamat,
48) 17aβ-Ethoxy-2-methoxy-17a,18a-dihomöstra-1,3,5(10)-trien-3-yl-sulfamat,
49) 2-Methoxy-17aβ-(n-propoxy)-17a,18a-dihomöstra-1,3,5(10)-trien-3-yl-sulfamat,
50) 2-Methoxy-17aß-methyl-17a,18a-dihomöstra-1,3,5(10)-trien-3-yl-sulfamat,
51) 17aβ-Difluormethyl-2-methoxy-17a,18a-dihomöstra-1,3,5(10)-trien-3-yl-sulfamat,
52) 17aβ-Fluormethyl-2-methoxy-17a,18a-dihomöstra-1,3,5(10)-trien-3-yl-sulfamat,
53)17aβ-Ethyl-2-methoxy-17a,18a-dihomöstra-1,3,5(10)-trien-3-yl-sulfamat,
54) 2-Methoxy-17a(20)-methylen-17a,18a-dihomöstra-1,3,5(10)-trien-3-yl-sulfamat,
55) 17a(20)-Difluormethylen-2-methoxy-17a,18a-dihomöstra-1,3,5(10)-trien-3-yl-sulfamat,
56) 17a(20)-Fluormethylen-2-methoxy-17a,18a-dihomöstra-1,3,5(10)-trien-3-yl-sulfamat,
57) 2-Ethyl-17a-oxo-17a-homöstra-1,3,5(10)-trien-3-yl-sulfamat,
58) 2-Ethyl-17a-oxo-17a-homöstra-1,3,5(10)-trien-3-yl(N-Acetyl)-sulfamat,
59) 2-Ethyl-17a-homöstra-1,3,5(10)-trien-3-yl-sulfamat,
60) 2-Ethyl-17a-homöstra-1,3,5(10)-trien-3-yl(N-Acetyl)-sulfamat,
61) 2-Ethyl-17aβ-hydroxy-17a-homöstra-1,3,5(10)-trien-3-yl-sulfamat,
62)2-Ethyl-17a-homöstra-1,3,5(10)-trien-3,17aβ-diyl-bissulfamat,
63)2-Ethyl-17a-homöstra-1,3,5(10)-trien-3,17aβ-diyl-bis(N-acetyl)-sulfamat,
64) 2-Ethyl-17aβ-methoxy-17a-homöstra-1,3,5(10)-trien-3-yl-sulfamat,
65) 2-Ethyl-17aβ-ethoxy-17a-homöstra-1,3,5(10)-trien-3-yl-sulfamat.

11. 2-Substituierte D-Homöstra-1,3,5(10)-trien-3-yl-sulfamate, wie definiert in einem der Ansprüche 1 bis 10, für die Verwendung als Medikament.

12. Verwendung der 2-Substituierten D-Homöstra-1,3,5(10)-trien-3-yl-sulfamate, wie definiert in einem der Ansprüche 1 bis 10, für die Herstellung eines Medikaments zur Behandlung von Tumorerkrankungen.

13. Verwendung nach Anspruch 12, wobei die Tumorerkrankungen durch die Inhibierung der Tubulinpolymerisation positiv beeinflußt werden können.

14. Verwendung nach Anspruch 12 oder 13, wobei wenigstens eine zusätzliche Wirkstoffkomponente für die Herstellung eines Medikaments verwendet wird.

15. Verwendung nach Anspruch 12, wobei die Tumorerkrankungen Tumorerkrankungen der männlichen und weiblichen Gonaden und der männlichen und weiblichen Sexualorgane sind.

16. Verwendung nach Anspruch 15, bei der der Tumor eine Tumorerkrankung der Brustdrüsen sind.

17. Verwendung der 2-Substituierten D-Homöstra-1,3,5(10)-trien-3-yl-sulfamate, wie definiert_in einem der Ansprüche 1 bis 10, für die Herstellung eines Medikaments zur Behandlung von Brustkrebs.

18. Verwendung der 2-Substituierten D-Homöstra-1,3,5(10)-trien-3-yl-sulfamate, wie definiert in einem der Ansprüche 1 bis 10, für die Herstellung eines Medikaments zur Behandlung von Prostatakrebs.

19. Pharmazeutische Zubereitungen, die wenigstens eine Verbindung der allgemeinen Formel I nach einem der Ansprüche 1 bis 10 zusammen mit pharmazeutisch verträglichen Adjuvantien und/oder Vehikelflüssigkeiten enthalten.

20. Pharmazeutische Zubereitungen nach Anspruch 19, umfassend wenigstens eine zusätzliche Wirkstoffkomponente.

## Claims

1. 2-Substituted D-homoestra-1,3,5(10)-trien-3-yl sulfamates of general formula I 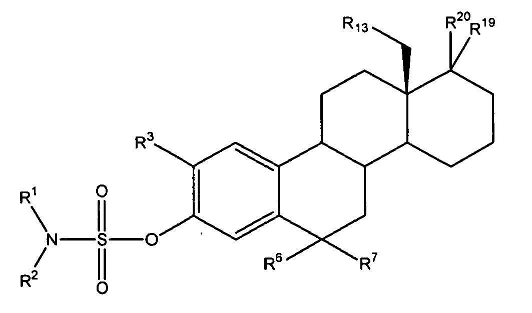 in which
R¹ and R², independently of one another, mean a hydrogen atom, a methyl group, a C₁-C₄-acyl group or a benzoyl group,
R³ means a C₁-C₅-alkyl, a C₁-C₅-alkyloxy group or a radical -O-CₙFₘHₒ, whereby n=1, 2, 3, 4, 5 or 6, m>1, and m+o=2n+1,
R⁶ and R⁷, independently of one another, mean a hydrogen atom, a hydroxy group, an amino group or an NHR⁸ group, wherein
R⁸ is an acetyl group, or
R⁶ and R⁷ together are an oxime NOH,
R¹³ is a hydrogen atom or a methyl group,
R¹⁹ is a hydrogen atom or a fluorine atom,
R²⁰ is a hydrogen atom or a fluorine atom or a hydroxy group or C₁-C₅-alkyloxy group or a C₁-C₅-alkyl group or a radical -CₙFₘHₒ, wherein n=1, 2, 3, 4, 5 or 6, m>1 and m+o=2n+1
or a group -OSO₂NR¹R²,
R¹⁹ and R²⁰ together mean an oxygen atom, a methylene group, a difluoromethylene group or a monofluoromethylene group or an oxime NOR²¹, wherein R²¹ is a hydrogen atom or a C₁-C₅-alkyl group,
as well as their pharmaceutically acceptable salts.

2. 2-Substituted D-homoestra-1,3,5(10)-trien-3-yl sulfamates according to claim 1, **characterized in that** R³ represents a methyl, ethyl, methoxy, ethoxy or 2,2,2-trifluoroethoxy group.

3. 2-Substituted D-homoestra-1,3,5(10)-trien-3-yl sulfamates according to claim 2, wherein R³ represents a methoxy group.

4. 2-Substituted D-homoestra-1,3,5(10)-trien-3-yl sulfamates according to claims 1 to 3, wherein R⁶ and R⁷ in each case represent a hydrogen atom.

5. 2-Substituted D-homoestra-1,3,5(10)-trien-3-yl sulfamates according to claims 1 to 4, wherein R¹⁹ represents a hydrogen atom.

6. 2-Substituted D-homoestra-1,3,5(10)-trien-3-yl sulfamates according to claims 1 to 5, wherein R²⁰ represents a hydrogen atom or a hydroxy group.

7. 2-Substituted D-homoestra-1,3,5(10)-trien-3-yl sulfamates according to claims 1 to 6, wherein R¹ represents a hydrogen atom.

8. 2-Substituted D-homoestra-1,3,5(10)-trien-3-yl sulfamates according to claims 1 to 7, wherein R² represents a hydrogen atom or an acyl group.

9. 2-Substituted D-homoestra-1,3,5(10)-trien-3-yl sulfamates according to claims 1 to 8, wherein R¹³ represents a hydrogen atom or a methyl group.

10. 2-Substituted D-homoestra-1,3,5(10)-trien-3-yl sulfamates according to claim 1, namely
1) 2-Methoxy-17a-oxo-17a-homoestra-1,3,5(10)-trien-3-yl sulfamate
2) 2-Methoxy-17a-oxo-17a-homoestra-1,3,5(10)-trien-3-yl (N-acetyl)-sulfamate
3) 2-Methoxy-17a-homoestra-1,3,5(10)-trien-3-yl sulfamate
4) 2-Methoxy-17a-homoestra-1,3,5(10)-trien-3-yl (N-acetyl)-sulfamate
5) 2-Methoxy-6-oximino-17a-homoestra-1,3,5(10)-trien-3-yl sulfamate
6) 2-Methoxy-6-(O-methyloximino)-17a-homoestra-1,3,5(10)-trien-3-yl sulfamate
7) 6α-Hydroxy-2-methoxy-17a-homoestra-1,3,5(10)-trien-3-yl sulfamate
8) 6a-Acetylamino-2-methoxy-17a-homoestra-1,3,5(10)-trien-3-yl sulfamate
9) 2-Methoxy-6-oxo-17a-homoestra-1,3,5(10)-trien-3-yl sulfamate
10) 17aα-Hydroxy-2-methoxy-17a-homoestra-1,3,5(10)-trien-3-yl sulfamate
11) 17aβ-Hydroxy-2-methoxy-17a-homoestra-1,3,5(10)-trien-3-yl sulfamate
12) 2-Methoxy-17a-homoestra-1,3,5(10)-triene-3,17aβ-diyl bissulfamate
13) 2-Methoxy-17a-homoestra-1,3,5(10)-triene-3,17aβ-diyl bis-(N-acetyl)-sulfamate
14) 17a-Difluoro-2-methoxy-17a-homoestra-1,3,5(10)-trien-3-yl sulfamate
15) 17aa-Fluoro-2-methoxy-17a-homoestra-1,3,5(10)-trien-3-yl sulfamate
16) 17aβ-Fluoro-2-methoxy-17a-homoestra-1,3,5(10)-trien-3-yl sulfamate
17) 2-Methoxy-17a-oximino-17a-homoestra-1,3,5(10)-trien-3-yl sulfamate
18) 2-Methoxy-17a-(methyloximino)-17a-homoestra-1,3,5(10)-trien-3-yl sulfamate
19) 2,17aβ-Dimethoxy-17a-homoestra-1,3,5(10)-trien-3-yl sulfamate
20) 2,17aβ-Dimethoxy-17a-homoestra-1,3,5(10)-trien-3-yl (N-acetyl)-sulfamate
21) 17aβ-Ethoxy-2-methoxy-17a-homoestra-1,3,5(10)-trien-3-yl sulfamate
22) 17aβ-Ethoxy-2-methoxy-17a-homoestra-1,3,5(10)-trien-3-yl (N-acetyl)-sulfamate
23) 2-Methoxy-17aβ-(n-propoxy)-17a-homoestra-1,3,5(10)-trien-3-yl sulfamate
24) 2-Methoxy-17aβ-methyl-17a-homoestra-1,3,5(10)-trien-3-yl sulfamate
25) 17aβ-Difluoromethyl-2-methoxy-17a-homoestra-1,3,5(10)-trien-3-yl sulfamate
26) 17aβ-Fluoromethyl-2-methoxy-17a-homoestra-1,3,5(10)-trien-3-yl sulfamate
27) 17aβ-Ethyl-2-methoxy-17a-homoestra-1,3,5(10)-trien-3-yl sulfamate
28) 2-Methoxy-17a(20)-methylene-17a-homoestra-1,3,5(10)-trien-3-yl sulfamate
29) 17a(20)-Difluoromethylene-2-methoxy-17a-homoestra-1,3,5(10)-trien-3-yl sulfamate
30) 17a(20)-Fluoromethylene-2-methoxy-17a-homoestra-1,3,5(10)-trien-3-yl sulfamate
31) 2-Methoxy-17a-oxo-17a,18a-dihomoestra-1,3,5(10)-trien-3-yl sulfamate
32) 2-Methoxy-17a-oxo-17a,18a-dihomoestra-1,3,5(10)-trien-3-yl (N-acetyl)-sulfamate
33) 2-Methoxy-17a,18a-dihomoestra-1,3,5(10)-trien-3-yl sulfamate
34) 2-Methoxy-17a,18a-dihomoestra-1,3,5(10)-trien-3-yl (N-acetyl)-sulfamate
35) 2-Methoxy-6-oximino-17a,18a-dihomoestra-1,3,5(10)-trien-3-yl sulfamate
36) 2-Methoxy-6-(O-methyloximino)-17a,18a-dihomoestra-1,3,5(10)-trien-3-yl sulfamate
37) 6α-Hydroxy-2-methoxy-17a,18a-dihomoestra-1,3,5(10)-trien-3-yl sulfamate
38) 6α-Acetylamino-2-methoxy-17a,18a-dihomoestra-1,3,5(10)-trien-3-yl sulfamate
39) 17aβ-Hydroxy-2-methoxy-17a,18a-dihomoestra-1,3,5(10)-trien-3-yl sulfamate
40) 2-Methoxy-17a, 18a-dihomoestra-1,3,5(10)-triene-3,17aβ-diyl bissulfamate
41) 2-Methoxy-17a,18a-dihomoestra-1,3,5(10)-triene-3,17aβ-diyl bis-(N-acetyl)-sulfamate
42) 17a-Difluoro-2-methoxy-17a,18a-dihomoestra-1,3,5(10)-trien-3-yl sulfamate
43) 17aα-Fluoro-2-methoxy-17a,18a-dihomoestra-1,3,5(10)-trien-3-yl sulfamate
44) 17aβ-Fluoro-2-methoxy-17a,18a-dihomoestra-1,3,5(10)-trien-3-yl sulfamate
45) 2-Methoxy-17a-oximino-17a,18a-dihomoestra-1,3,5 (10)-trien-3-yl sulfamate
46) 2-Methoxy-17a-(methyloximino)-17a,18a-dihomoestra-1,3,5(10)-trien-3-yl sulfamate
47) 2,17aβ-Dimethoxy-17a,18a-dihomoestra-1,3,5(10)-trien-3-yl sulfamate
48) 17aβ-Ethoxy-2-methoxy-17a,18a-dihomoestra-1,3,5(10)-trien-3-yl sulfamate
49) 2-Methoxy-17aβ-(n-propoxy)-17a,18a-dihomoestra-1,3,5(10)-trien-3-yl sulfamate
50) 2-Methoxy-17aβ-methyl-17a,18a-dihomoestra-1,3,5(10)-trien-3-yl sulfamate
51) 17aβ-Difluoromethyl-2-methoxy-17a,18a-dihomoestra-1,3,5(10)-trien-3-yl sulfamate
52) 17aβ-Fluoromethyl-2-methoxy-17a,18a-dihomoestra-1,3,5(10)-trien-3-yl sulfamate
53) 17aβ-Ethyl-2-methoxy-17a,18a-dihomoestra-1,3,5(10)-trien-3-yl sulfamate
54) 2-Methoxy-17a(20)-methylene-17a,18a-dihomoestra-1,3,5(10)-trien-3-yl sulfamate
55) 17a(20)-Difluoromethylene-2-methoxy-17a,18a-dihomoestra-1,3,5(10)-trien-3-yl sulfamate
56) 17a(20)-Fluoromethylene-2-methoxy-17a,18a-dihomoestra-1,3,5(10)-trien-3-yl sulfamate
57) 2-Ethyl-17a-oxo-17a-homoestra-1,3,5(10)-trien-3-yl sulfamate
58) 2-Ethyl-17a-oxo-17a-homoestra-1,3,5(10)-trien-3-yl (N-acetyl)-sulfamate
59) 2-Ethyl-17a-homoestra-1,3,5(10)-trien-3-yl sulfamate
60) 2-Ethyl-17a-homoestra-1,3,5(10)-trien-3-yl (N-acetyl)-sulfamate
61) 2-Ethyl-17aβ-hydroxy-17a-homoestra-1,3,5(10)-trien-3-yl sulfamate
62) 2-Ethyl-17a-homoestra-1,3,5(10)-triene-3,17aβ-diyl bissulfamate
63) 2-Ethyl-17a-homoestra-1,3,5(10)-triene-3,17aβ-diyl bis-(N-acetyl)-sulfamate
64) 2-Ethyl-17aβ-methoxy-17a-homoestra-1,3,5(10)-trien-3-yl sulfamate
65) 2-Ethyl-17aβ-ethoxy-17a-homoestra-1,3,5(10)-trien-3-yl sulfamate

11. 2-substituted D-homoestra-1,3,5(10)-trien-3-yl sulfamates as defined in any one of claims 1 to 10 for use as a medicament.

12. Use of 2-substituted D-homoestra-1,3,5(10)-trien-3-yl sulfamates as defined in any one of claims 1 to 10 for the production of a medicament for treating tumour diseases.

13. Use according to claim 12 wherein the tumour diseases can be influenced positively by the inhibition of tubulin polymerization.

14. Use according to claim 12 or 13 whereby at least one additional active ingredient is used for the production of a medicament.

15. Use according to claim 12 wherein the tumour diseases are tumour diseases of male and female gonads, male and female sex organs.

16. Use according to claim 15 wherein the tumour is tumour disease of the mammary glands.

17. Use of 2-substituted D-homoestra-1,3,5(10)-trien-3-yl sulfamates as defined in any one of claims 1 to 10 for the production of a medicament for treating breast cancer.

18. Use of 2-substituted D-homoestra-1,3,5(10)-trien-3-yl sulfamates as defined in any one of claims 1 to 10 for the production of a medicament for treating prostate cancer.

19. Pharmaceutical compositions that contain at least one compound of general formula I according to one of claims 1 to 10 together with pharmaceutically compatible adjuvants and/or vehicles.

20. Pharmaceutical compositions according to claim 19 comprising at least one additional active ingredient.

## Revendications

1. Sulfamates de D-homo-oestra-1,3,5(10)-triène-3-yle substitués en position 2, de formule générale I 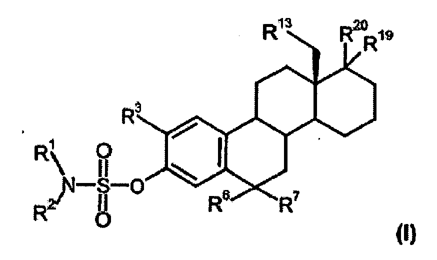 dans laquelle
R¹ et R² représentent, indépendamment l'un de l'autre, un atome de H, un groupe méthyle, un groupe acyle en C₁ à C₄ ou un groupe benzoyle,
R³ représente un groupe alkyle en C₁ à C₅ ou alkoxy en C₁ à C₅ ou un reste -O-CₙFₘHₒ, dans lequel n a la valeur 1, 2, 3, 4, 5 ou 6, m est supérieur à 1 et la somme m + o est égale à 2n + 1,
R⁶ et R⁷ représentent, indépendamment l'un de l'autre, un atome de H, un groupe hydroxy, amino ou NHR⁸, dans lequel
R⁸ représente un groupe acétyle, ou bien
R⁶ et R⁷ forment ensemble une oxime NOH,
R¹³ représente un atome de H ou un groupe méthyle,
R¹⁹ représente un atome de H ou un atome de F et
R²⁰ représente un atome de H ou un atome de F ou un groupe hydroxy, alkyloxy en C₁ à C₅ ou alkyle en C₁ à C₅, ou bien un reste -CₙFₘHₒ, dans lequel n a la valeur 1, 2, 3, 4, 5 ou 6, m est supérieur à 1 et la somme m + o est égale à 2n + 1, ou un groupe OSO₂NR¹R²,
R¹⁹ et R²⁰ forment ensemble un atome de O, un groupe méthylène, difluorométhylène ou monofluorométhylène, ou une oxime NOR²¹, dans laquelle
R²¹ représente un atome de H ou un groupe alkyle en C₁ à C₅, ainsi que leurs sels pharmaceutiquement acceptables.

2. Sulfamates de D-homo-oestra-1,3,5(10)-triène-3-yle substitués en position 2 suivant la revendication 1, **caractérisés en ce que** R³ représente un groupe méthyle, éthyle, méthoxy, éthoxy ou 2,2,2-trifluoréthoxy.

3. Sulfamates de D-homo-oestra-1,3,5(10)-triène-3-yle substitués en position 2 suivant la revendication 2, dans lesquels R³ représente un groupe méthoxy.

4. Sulfamates de D-homo-oestra-1,3,5(10)-triène-3-yle substitués en position 2 suivant les revendications 1 à 3, dans lesquels R⁶ et R⁷ représentent dans chaque cas un atome de H.

5. Sulfamates de D-homo-oestra-1,3,5(10)-triène-3-yle substitués en position 2 suivant les revendications 1 à 4, dans lesquels R¹⁹ représente un atome de H.

6. Sulfamates de D-homo-oestra-1,3,5(10)-triène-3-yle substitués en position 2 suivant les revendications 1 à 5, dans lesquels R²⁰ représente un atome de H ou un groupe hydroxy.

7. Sulfamates de D-homo-oestra-1,3,5(10)-triène-3-yle substitués en position 2 suivant les revendications 1 à 6, dans lesquels R¹ représente un atome de H.

8. Sulfamates de D-homo-oestra-1,3,5(10)-triène-3-yle substitués en position 2 suivant les revendications 1 à 7, dans lesquels R² représente un atome de H ou un groupe acyle.

9. Sulfamates de D-homo-oestra-1,3,5(10)-triène-3-yle substitués en position 2 suivant les revendications 1 à 8, dans lesquels R¹³ représente un atome de H ou un groupe méthyle.

10. Sulfamates de D-homo-oestra-1,3,5(10)-triène-3-yle substitués en position 2 suivant la revendication 1, à savoir :
1) sulfamate de 2-méthoxy-17a-oxo-17a-homo-oestra-1,3,5(10)triène-3-yle,
2) (N-acétyl)-sulfamate de 2-méthoxy-17a-oxo-17a-homooestra-1,3,5(10)triène-3-yle,
3) sulfamate de 2-méthoxy-17a-homo-oestra-1,3,5(10)-triène-3-yle,
4) (N-acétyl)-sulfamate de 2-méthoxy-17a-homo-oestra-1,3,5(10)triène-3-yle,
5) sulfamate de 2-méthoxy-6-oximino-17a-homo-oestra-1,3,5(10)triène-3-yle,
6) sulfamate de 2-méthoxy-6-(O-méthyloximino)-17a-homo-oestra-1,3,5(10)triène-3-yle,
7) sulfamate de 6α-hydroxy-2-méthoxy-17a-homo-oestra-1,3,5(10)triène-3-yle,
8) sulfamate de 6α-acétylamino-2-méthoxy-17a-homo-oestra-1,3,5(10)triène-3-yle,
9) sulfamate de 2-méthoxy-6-oxo-17a-homo-oestra-1,3,5(10)-triène-3-yle,
10) sulfamate de 17aα-hydroxy-2-méthoxy-17a-homo-oestra-1,3,5(10)triène-3-yle,
11) sulfamate de 17aβ-hydroxy-2-méthoxy-17a-homo-oestra-1,3,5(10)triène-3-yle,
12) bis-sulfamate de 2-méthoxy-17a-homo-oestra-1,3,5(10)-triène-3,17αβ-diyle,
13) bis-(N-acétyl)-sulfamate de 2-méthoxy-17a-homooestra-1,3,5(10)-triène-3,17αβ-diyle,
14) sulfamate de 17a-difluoro-2-méthoxy-17a-homo-oestra-1,3,5(10)-triène-3-yle,
15) sulfamate de 17aα-fluoro-2-méthoxy-17a-homo-oestra-1,3,5(10)-triène-3-yle,
16) sulfamate de 17aβ-fluoro-2-méthoxy-17a-homo-oestra-1,3,5(10)-triène-3-yle,
17) sulfamate de 2-méthoxy-17a-oximino-17a-homo-oestra-1,3,5(10)-triène-3-yle,
18) sulfamate de 2-méthoxy-17a-(méthyloximino)-17a-homooestra-1,3,5(10)-triène-3-yle,
19) sulfamate de 2,17aβ-diméthoxy-17a-homo-oestra-1,3,5(10)-triène-3-yle,
20) (N-acétyl)-sulfamate de 2,17aβ-diméthoxy-17a-homooestra-1,3,5(10)-triène-3-yle,
21) sulfamate de 17aβ-éthoxy-2-méthoxy-17a-homo-oestra-1,3,5(10)-triène-3-yle,
22) (N-acétyl)-sulfamate de 17aβ-éthoxy-2-méthoxy-17a-homo-oestra-1,3,5(10)-triène-3-yle,
23) sulfamate de 2-méthoxy-17aβ-(n-propoxy)-17a-homooestra-1,3,5(10)-triène-3-yle,
24) sulfamate de 2-méthoxy-17aβ-méthyl-17a-homo-oestra-1,3,5(10)-triène-3-yle,
25) sulfamate de 17aβ-difluorométhyl-2-méthoxy-17a-homo-oestra-1,3,5(10)-triène-3-yle,
26) sulfamate de 17aβ-fluorométhyl-2-méthoxy-17a-homo-oestra-1,3,5(10)-triène-3-yle,
27) sulfamate de 17aβ-éthyl-2-méthoxy-17a-homo-oestra-1,3,5(10)-triène-3-yle,
28) sulfamate de 2-méthoxy-17a(20)-méthylène-17a-homooestra-1,3,5(10)-triène-3-yle,
29) sulfamate de 17a(20)-difluorométhylène-2-méthoxy-17a-homo-oestra-1,3,5(10)-triène-3-yle,
30) sulfamate de 17a(20)-fluorométhylène-2-méthoxy-17a-homo-oestra-1,3,5(10)-triène-3-yle,
31) sulfamate de 2-méthoxy-17a-oxo-17a,18a-dihomooestra-1,3,5(10)-triène-3-yle,
32) (N-acétyl)-sulfamate de 2-méthoxy-17a-oxo-17a,18a-dihomo-oestra-1,3,5(10)-triène-3-yle,
33) sulfamate de 2-méthoxy-17a,18a-dihomo-oestra-1,3,5(10)-triène-3-yle,
34) (N-acétyl)-sulfamate de 2-méthoxy-17a,18a-dihomooestra-1,3,5(10)-triène-3-yle,
35) sulfamate de 2-méthoxy-6-oximino-17a,18a-dihomooestra-1,3,5(10)-triène-3-yle,
36) sulfamate de 2-méthoxy-6-(O-méthyloximino)-17a,18a-dihomo-oestra-1,3,5(10)-triène-3-yle,
37) sulfamate de 6α-hydroxy-2-méthoxy-17a,18a-dihomooestra-1,3,5(10)-triène-3-yle,
38) sulfamate de 6α-acétylamino-2-méthoxy-17a,18a-dihomooestra-1,3,5(10)-triène-3-yle,
39) sulfamate de 17aβ-hydroxy-2-méthoxy-17a,18a-dihomooestra-1,3,5(10)-triène-3-yle,
40) bis-sulfamate de 2-méthoxy-17a,18a-dihomo-oestra-1,3,5(10)-triène-3,17aβ-diyle,
41) (N-acéty)-bis-sulfamate de 2-méthoxy-17a,18a-dihomooestra-1,3,5(10)-triène-3,17aβ-diyle,
42) sulfamate de 17a-difluoro-2-méthoxy-17a,18a-dihomooestra-1,3,5(10)-triène-3-yle,
43) sulfamate de 17aα-fluoro-2-méthoxy-17a,18a-dihomooestra-1,3,5(10)-triène-3-yle,
44) sulfamate de 17aβ-fluoro-2-méthoxy-17a,18a-dihomooestra-1,3,5(10)-triène-3-yle,
45) sulfamate de 2-méthoxy-17a-oximino-17a,18a-dihomooestra-1,3,5(10)-triène-3-yle,
46) sulfamate de 2-méthoxy-17a-(méthyloximino)-17a,18a-dihomo-oestra-1,3,5(10)-triène-3-yle,
47) sulfamate de 2,17aβ-diméthoxy-17a,18a-dihomo-oestra-1,3,5(10)-triène-3-yle,
48) sulfamate de 17aβ-éthoxy-2-méthoxy-17a,18a-dihomooestra-1,3,5(10)-triène-3-yle,
49) sulfamate de 2-méthoxy-17aβ-(n-propoxy)-17a,18a-dihomo-oestra-1,3,5(10)-triène-3-yle,
50) sulfamate de 2-méthoxy-17aβ-méthyl-17a,18a-dihomooestra-1,3,5(10)-triène-3-yle,
51) sulfamate de 17aβ-difluorométhyl-2-méthoxy-17a,18a-dihomo-oestra-1,3,5(10)-triène-3-yle,
52) sulfamate de 17aβ-fluorométhyl-2-méthoxy-17a,18a-dihomo-oestra-1,3,5(10)-triène-3-yle,
53) sulfamate de 17aβ-éthyl-2-méthoxy-17a,18a-dihomooestra-1,3,5(10)-triène-3-yle,
54) sulfamate de 2-méthoxy-17a(20)-méthylène-17a,18a-dihomo-oestra-1,3,5(10)-triène-3-yle,
55) sulfamate de 17a(20)-difluorométhylène-2-méthoxy-17a,18a-dihomo-oestra-1,3,5(10)-triène-3-yle,
56) sulfamate de 17a(20)-fluorométhylène-2-méthoxy-17a,18a-dihomo-oestra-1,3,5(10)-triène-3-yle,
57) sulfamate de 2-éthyl-17a-oxo-17a-homo-oestra-1,3,5(10)-triène-3-yle,
58) (N-acétyl)-sulfamate de 2-éthyl-17a-oxo-17a-homooestra-1,3,5(10)-triène-3-yle,
59) sulfamate de 2-éthyl-17a-homo-oestra-1,3,5(10)-triène-3-yle,
60) (N-acétyl)-sulfamate de 2-éthyl-17a-homo-oestra-1,3,5(10)-triène-3-yle,
61) sulfamate de 2-éthyl-17aβ-hydroxy-17a-homo-oestra-1,3,5(10)-triène-3-yle,
62) bis-sulfamate de 2-éthyl-17a-homo-oestra-1,3,5(10)-triène-3,17aβ-diyle,
63) bis-(N-acétyl)-sulfamate de 2-éthyl-17a-homo-oestra-1,3,5(10)-triène-3,17aβ-diyle,
64) sulfamate de 2-éthyl-17aβ-méthoxy-17a-homo-oestra-1,3,5(10)-triène-3-yle,
65) sulfamate de 2-éthyl-17aβ-éthoxy-17a-homo-oestra-1,3,5(10)-triène-3-yle.

11. Sulfamates de D-homo-oestra-1,3,5(10)-triène-3-yle substitués en position 2, tels que définis dans l'une des revendications 1 à 10, destinés à l'utilisation comme médicament.

12. Utilisation des sulfamates de D-homo-oestra-1,3,5(10)-triène-3-yle substitués en position 2, tels que définis dans l'une des revendications 1 à 10, pour la préparation d'un médicament destiné au traitement de maladies tumorales.

13. Utilisation suivant la revendication 12, dans laquelle les maladies tumorales peuvent être influencées positivement par l'inhibition de la polymérisation de tubuline.

14. Utilisation suivant la revendication 12 ou 13, dans laquelle au moins un composant actif supplémentaire est utilisé pour la préparation d'un médicament.

15. Utilisation suivant la revendication 12, dans laquelle les maladies tumorales sont des maladies tumorales affectant les gonades mâles et femelles et les organes sexuels masculins et féminins.

16. Utilisation suivant la revendication 15, dans laquelle la tumeur est une maladie tumorale des glandes mammaires.

17. Utilisation des sulfamates de D-homo-oestra-1,3,5(10)-triène-3-yle substitués en position 2, tels que définis dans l'une des revendications 1 à 10, pour la préparation d'un médicament destiné au traitement du cancer du sein.

18. Utilisation des sulfamates de D-homo-oestra-1,3,5(10)-triène-3-yle substitués en position 2, tels que définis dans l'une des revendications 1 à 10, pour la préparation d'un médicament destiné au traitement du cancer de la prostate.

19. Préparations pharmaceutiques qui contiennent au moins un composé de formule générale I suivant l'une des revendications 1 à 10 conjointement avec des adjuvants et/ou des véhicules liquides pharmaceutiquement acceptables.

20. Préparations pharmaceutiques suivant la revendication 19, comprenant au moins un composant actif supplémentaire.
